# EUROPEAN PATENT SPECIFICATION

(11) **EP 0 889 898 B1**
(45) Date of publication and mention of the grant of the patent: **20.02.2002**
(21) Application number: 97929243.0
(22) Date of filing: 20.06.1997
(51) Int. Cl.: C07H 15/252, A61K 31/70

(54) **MORPHOLINYL ANTHRACYCLINE DERIVATIVES**
MORPHOLINYL-ANTHRACYCLINDERIVATE
DERIVES DE LA MORPHOLINYLE ANTHRACYCLINE

(30) Priority: 11.07.1996 GB 9614621
(43) Date of publication of application: 13.01.1999
(73) Proprietor: PHARMACIA & UPJOHN S.p.A., 20152 Milano (IT)
(72) Inventor: CARUSO, Michele, I-20131 Milan (IT); LOVISOLO, Pierpaolo, I-20144 Milan (IT); GERONI, Cristina, I-20149 Milan (IT); SUARATO, Antonino, I-20158 Milan (IT)
(86) International application number: EP9703252
(87) International publication number: WO9802446

(56) References cited:
- EP-A- 0 206 138
- EP-A- 0 239 774
- EP-A- 0 376 177
- GB-A- 2 157 290
- US-A- 4 826 964

## Description

The present invention relates to morpholinyl anthracycline derivatives, to a process for their preparation and to pharmaceutical compositions containing them.

The morpholinyl anthracyclines are semisynthetic analogs of anthracyclines endowed with remarkable antitumor activity (see: Bioactive Molecules Vol. 6, Ed. J.W. Lown, Elviser 1988). At present, several morpholinyl anthracyclines are under clinical evaluation. Among them, 3'-deamino-3'-[2''(S) -methoxy-4"-morpholinyl] doxorubicin (1) possesses high *in vitro* and *in vivo* activity on sensitive or multidrug resistant tumors including doxorubicin-resistant tumor cells (see: M. Grandi *et al*., Cancer Treat. Rew., 17, 133, 1990 and M. Ripamonti *et al*., Br. J. Cancer, 65, 703, 1992).

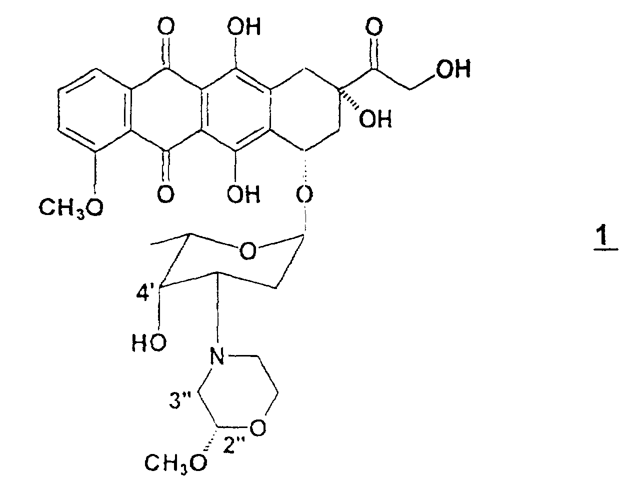

In US-A-4,826,964 (1989), E.D. Acton has described the preparation of bridged oxygen derivatives of dauncrubicin and doxorubicin. US-A-4,826,964 also describes compounds of general formula A: 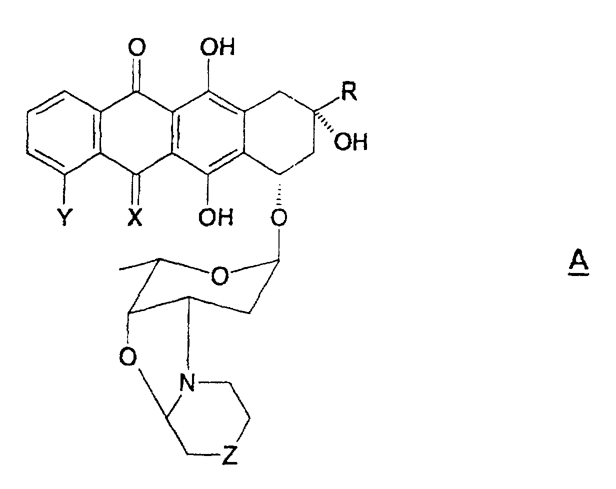 in which each of X and Z is an oxygen atom, Y is a methoxy group and R is the residue -COCH₂OH. Compounds of formula A were prepared in small amounts by reductive alkylation of anthracyclines with a dialdehyde of formula:

HOC-(CH₂)₂-O-(CH₂)₂-CHO

in the presence of reducing agents such as sodium or potassium cyanoborohydride.

The present invention relates to new derivatives of morpholinyl anthracyclines in which the morpholino ring is bridged with an oxygen atom to the position C-4' of the sugar residue. The present invention provides a morpholinyl anthracycline derivative of formula 2: 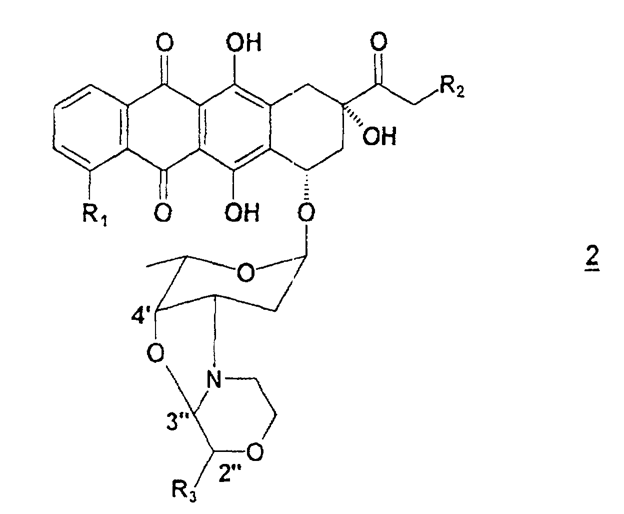 wherein: R₁ is methoxy, hydroxy or hydrogen, R₂ is hydrogen or hydroxy, R₃ is a C₁-C₅ alkoxy group, and the chiral carbon atom configurations at the 2" and 3" positions are 2''(S), 3''(R) or 2''(R), 3''(S); or a pharmaceutically acceptable salt thereof. R³ may be methoxy, ethoxy, propoxy, butoxy or pentoxy, preferably methexy. R¹ is preferably methoxy. R² is preferably hydroxy. The invention also provides a process for the preparation of compounds of formula 2, pharmaceutical compositions containing them and the use thereof in treating certain mammalian diseases.

Compounds of formula (2), 3'-deamino-3",4'-anhydro-[2"-alkoxy-3"-hydroxy-4"-morpholinyl] anthracycline derivatives, show higher cytotoxic efficacy compared to that of the parent morpholinyl anthracyclines of formula 3: 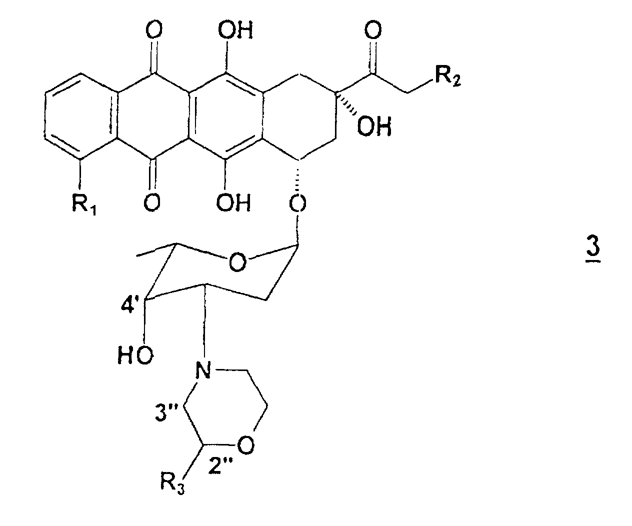 wherein R₁, R₂, R₃ and tne configuration at C-2" are as above defined.

The present invention also provides a process for preparing a compound of formula 2 or a pharmaceutically acceptable salt thereof, which comprises
(a) reacting an N-oxide anthracycline derivative of formula (4): 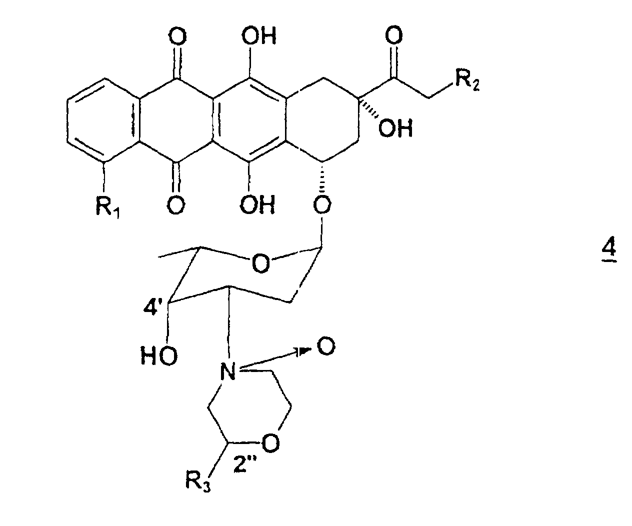 wherein R₁, R₂, R₃ and the configuration at C-2" are as above defined, with an iron (II) salt in presence of an iron-complexing agent, and
(b) if necessary converting the resultant compound of formula 2 into a pharmaceutically acceptable salt thereof.

It should be stressed that the presence of iron-complexing agent is crucial for the formation of compound 2. The reaction proceeds with formation of single isomer and formation of the parent morpholinyl anthracycline of formula 3, which may be recovered. Preferably, the iron salt is iron (II) chloride tetrahydate. Preferably, the complexing agent is tartaric acid. Preferably, the reaction is performed in water. The reaction can be carried out at room temperature. Typically, the reaction time is 48 hours.

The starting compounds of formula 4 may be prepared as described in GB-A-2296495.

The new anthracycline derivatives of the present invention are endowed with antitumoral activity.

The present invention also provides a pharmaceutical composition comprising a pharmaceutically acceptable diluent or carrier and, as an active principle, a morpholinyl anthracycline compound of formula 2 or a pharmaceutically acceptable salt thereof.

Suitable routes of administration include parenteral administration. For parenteral administration a liquid formulation may be prepared using the active compound and a sterile diluent or carrier which may either dissolve the active compound or provide a suspension for it. The parenteral formulation may be prepared in the form of a sterile solid for reconstitution prior to administration with a suitable vehicle such as physiological saline, sterile water or other sterile vehicle.

The compounds of the invention are useful in methods of treatment of leukaemia or colon adenocarcinoma. A therapeutically effective amount is administered to a patient having a tumor to ameliorate or improve the condition of the patient. An amount sufficient to inhibit the growth of the tumor may be administered. The dosage to be given can be ascertained using known dosage ranges for doxorubicin and daunorubicin modified by reference to the activity shown by the present compounds in *in vitro* and *in vivo* anti-tumor tests. Suitable dosages are generally in the range of from 0.01 to 100 mg/m² body surface, preferably from 0.1 to 10 mg/m², depending on the nature and severity of the disease being treated and on the general condition of the patient.

The following Examples further illustrate the present invention.

### Example 1: Preparation of 3'-deamino-3"-4'-anhydro-[2"(S)-methoxy-3"(R)-hydroxy-4"-morpholinyl]doxorubicin (2a)

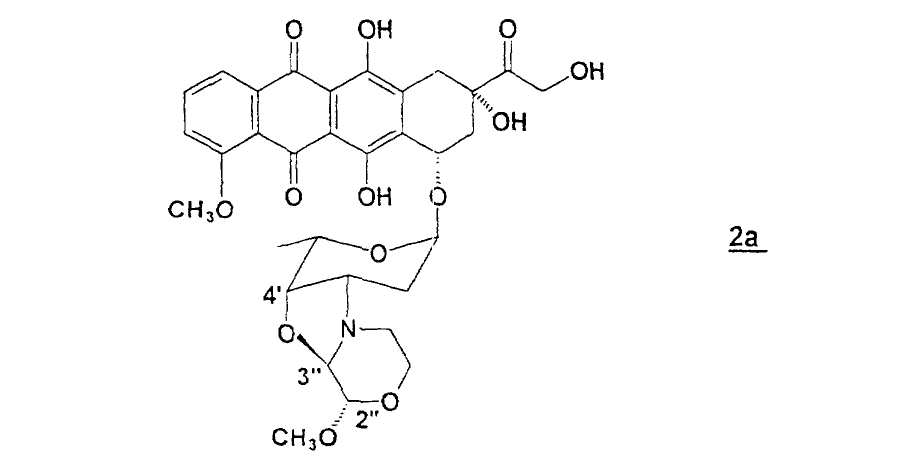

3'deamino-3'-[2"(S)-methoxy-4"-morpholinyl]-3'-N-oxide-doxorubicin (1, 0.16g, 0.234mmol) is dissolved in a water solution (10ml) containing iron (II) chloride tetrahydrate (0.11g) and tartaric acid (0.75g). The mixture is kept under stirring, in the dark at room temperature, for 48 hours. After that, methylene chloride (50ml) is added and the crude product is extracted in the organic phase from which the title compound 2a is separated after flash chromatography on silica gel (eluting system: methylene chloride/ethanol 24/1 v/v). Yield 10 mg. TLC on kieselgel plate F₂₅₄ (Merck), eluting system methylene chloride/methanol (9/1 v/v), R_{f}=0.62. FD-MS: E.H.C. 30mA; m/z 642 (93, [MH]+); m/z 641 (100, [M]+). The structure of compound 2a was assigned on the basis of ¹HNMR studies.
¹HNMR (400 MHz, CDCl₃) δ:
1.38 (d, J=6.4Hz, 3H, CH₃-5'); 1.72 (ddd, J=6.2, 6.2, 15.4Hz, 1H, H-2'β); 2.01 (ddd, J=5.5, 5.5, 15.4Hz, 1H, H -2'α); 2.10 (dd, J=3.8, 14.9Hz, 1H, H-8az); 2.49 (ddd, J=1.7, 2.1, 14.9Hz, 1H, H-8eq); 2.73 (m, 1H, H-4"ax); 2.76 (m, 1H, H-2"eq); 3.00 (t, J=3.8Hz, 1H, CH₂-OH) ; 3.05 (d, J=19.2Hz, 1H, H-10ax) ; 3.25 (dd, J=1.7, 19.2Hz, 1H, H-10eq); 3.37 (m, 1H, H-3'); 3.45 (s, 3H, OCH₃-2"); 3.58 (m, 1H, H-5"eq); 3.91 (m, 1H, H-5"ax); 3.99 (dq, J=1.7, 6.4Hz, 1H, H-5'); 4.08 (dd, J=1.7, 6.4Hz, 1H, H-4'); 4.08 (s, 3H, OCH₃-4); 4.45 (d, J=2.1Hz, 1H, H-3"); 4.69 (d, J=2.1Hz, 1H, H-2"); 4.75 (d, J=3.8Hz, 2H, CH₂OH); 4.87 (s, 1H, OH-9); 5.31 (dd, J=2.1, 3.8Hz, 1H, H-7); 5.47 (dd, J=5.5, 6.2Hz, 1H, H-1'); 7.39 (d, J=7.7Hz, 1H, H-3); 7.78 (dd, J=7.3, 7.7Hz, H-2); 8.02 (d, J=7.3Hz, 1H, H-1); 13.25 (s, 1H, OH-11); 13.99 (s, 1H, OH-6).

### Example 2: Biological Activity

3'-Deamino-3",4'-anhydro-[2"(S)-methoxy-3"(R)-hydroxy-4"-morpholinyl]doxorubicin 2a was tested *in vitro* on L1210 leukemia cells in comparison with 3'-deamino-3'-[2"(S)-methoxy-4"-morpholinyl]doxorubicin (1). The cytotoxic activity is reported as IC₅₀, the concentration inhibiting 50% of colony growth, calculated on concentration response curves. Compound 2a was found 2.5x10³ fold more potent than compound 1 (Table 1).

**Table 1:**

| *In vitro* cytotoxic activity (IC₅₀) of 3'-deamino-3",4'-anhydro-[2"(S)-methoxy-3"-hydroxy-4"-morpholinyl]doxorubicin (2a) and 3'-deamino-3'-[2"(S)-methoxy-4"-morpholinyl]doxorubicin (1) on L1210 leukemia cells. | |
|---|---|
| Growth inhibition test: 48h treatment | |
| Compound | IC₅₀ |
| 2a | 0.000292 ± 0.00008 |
| 1 | 7.38 ± 1 |

## Claims

1. A morpholinyl derivative of formula 2: 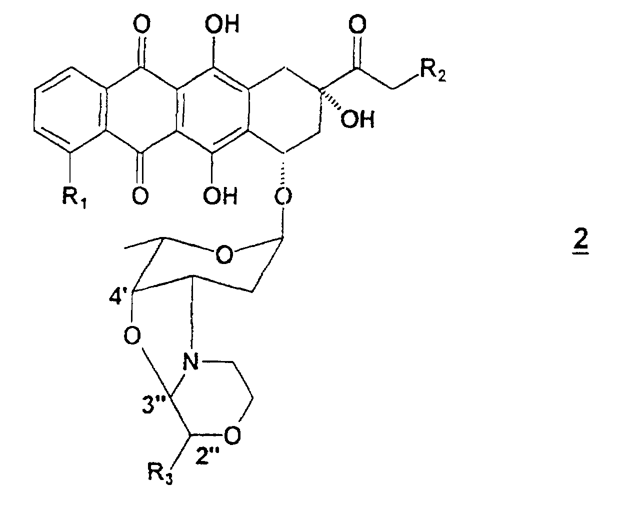 wherein: R₁ is methoxy, hydroxy or hydrogen, R₂ is hydrogen or hydroxy, R₃ is a C₁-C₅ alkoxy group, and the chiral carbon atom configurations at the 2" and 3" positions are 2"(S), 3"(R) or 2"(R), 3"(S); or a pharmaceutically acceptable salt thereof.

2. A compound according to claim 1 wherein R₂ is hydroxy.

3. A compound according to claim 1 or 2 wherein R₁ is methoxy.

4. A compound according to any one of the preceding claims wherein R₃ is methoxy.

5. A compound according to any one of claims 1 to 4 which is 3'-deamino-3",4'-anhydro-[2"(S)-methoxy-3"(R)-hydroxy-4"-morpholinyl] doxorubicin.

6. A process for the preparation of a compound as defined in claim 1, which comprises
(a) reacting a N-oxide anthraycline derivative of formula 4: 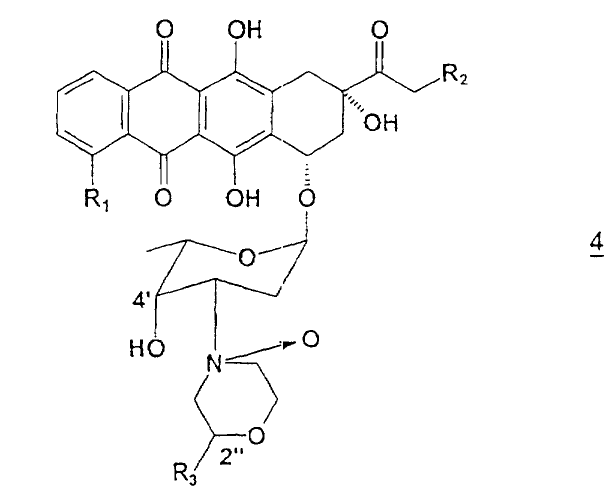 wherein R₁, R₂, R₃ and the configuration at C-2" are as defined in claim 1, with an iron (II) salt in presence of an iron complexing agent, and
(b) if necessary converting the resultant morpholinyl derivative of formula 2 into a pharmaceutically acceptable salt thereof.

7. A process according to claim 6, wherein step (a) is carried out in water, the iron salt is iron (II) chloride tetrahydrate and the complexing agent is tartaric acid.

8. A pharmaceutical composition comprising a pharmaceutically acceptable diluent or carrier and, as an active principle, a morpholinyl derivative of formula (2), or a pharmaceutically acceptable salt thereof, as defined in any one of claims 1 to 5.

9. A morpholinyl derivative of formula 2, or a pharmaceutically acceptable salt thereof, as defined in any one of claims 1 to 5 for use in a method of treatment of the human or animal body by therapy.

10. A morpholinyl derivative of formula 2, or a pharmaceutically acceptable salt thereof, as defined in any one of claims 1 to 5 for use as an anti-tumor agent.

## Patentansprüche

1. Morpholinylderivat der Formel (2): 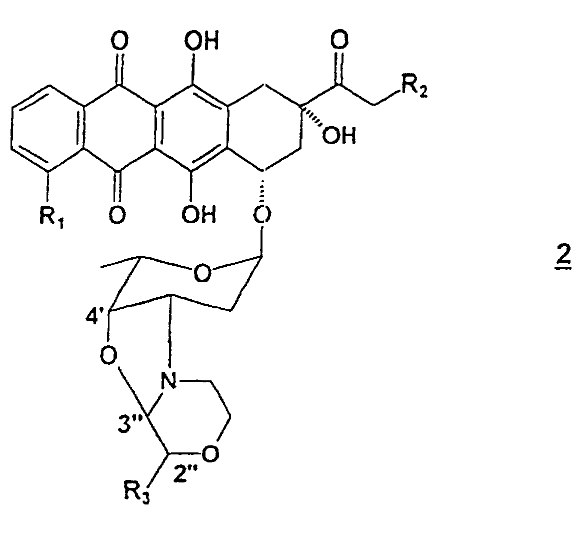 worin: R₁ Methoxy, Hydroxy oder Wasserstoff ist, R₂ Wasserstoff oder Hydroxy ist, R₃ eine C₁-C₅-Alkoxygruppe ist, und die Konfigurationen der chiralen Kohlenstoffatome in den 2''- und 3''-Positionen 2''(S), 3''(R), oder 2''(R), 3''(S) sind; oder ein pharmazeutisch annehmbares Salz davon.

2. Verbindung gemäß Anspruch 1, worin R₂ Hydroxy ist.

3. Verbindung gemäß Ansprach 1 oder 2, worin R₁ Methoxy ist.

4. Verbindung gemäß einem der vorangehenden Ansprüche, worin R₃ Methoxy ist.

5. Verbindung gemäß einem der Ansprüche 1 bis 4, die 3'-Deamino-3'',4'-anhydro-[2''(S)-methoxy-3''(R)-hydroxy-4''-morpholinyl]doxorubicin ist.

6. Verfahren zur Herstellung einer Verbindung gemäß Anspruch 1, das umfaßt:
(a) Umsetzen eines N-Oxidanthracyclinderivats der Formel (4): 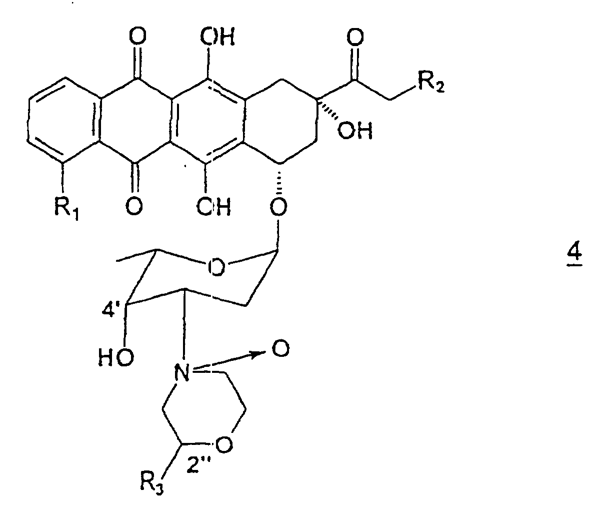 worin R₁, R₂, R₃ und die Konfiguration an C-2'' wie in Anspruch 1 definiert sind, mit einem Eisen(II)-Salz in der Gegenwart eines Eisenkomplexbildners, und
(b) falls notwendig Umwandeln des resultierenden Morpholinylderivats der Formel (2) in ein pharmazeutisch annehmbares Salz davon.

7. Verfahren gemäß Anspruch 6, worin Schritt (a) in Wasser durchgeführt wird, das Eisensalz Eisen(II)chloridtetrahydrat ist und der Komplexbildner Weinsäure ist.

8. Pharmazeutische Zusammensetzung umfassend ein pharmazeutisch annehmbares Diluens oder Träger und, als einen aktiven Wirkstoff, ein Morpholinylderivat der Formel (2), oder ein pharmazeutisch annehmbares Salz davon, gemäß einem der Ansprüche 1 bis 5.

9. Morpholinylderivat der Formel (2), oder ein pharmazeutisch annehmbares Salz davon, gemäß einem der Ansprüche 1 bis 5 zur Verwendung in einem Verfahren zur Behandlung des menschlichen oder tierischen Körpers in einer Therapie.

10. Morpholinylderivat der Formel (2) oder ein pharmazeutisch annehmbares Salz davon gemäß einem der Ansprüche 1 bis 5 zur Verwendung als ein Antitumormittel.

## Revendications

1. Dérivé morpholinyle de formule 2 : 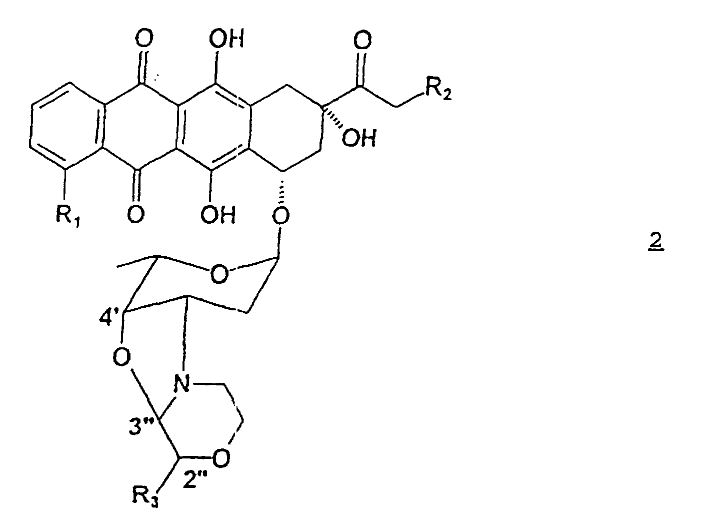 dans laquelle R₁ est méthoxy, hydroxy ou hydrogène, R₂ est hydrogène ou hydroxy, R₃ est un groupe alkoxy en C₁-C₅ et les configurations des atomes de carbone chiraux en positions 2'' et 3'' sont 2''(S), 3''(R) ou 2''(R), 3''(S) ; ou un sel pharmaceutiquement acceptable de celui-ci.

2. Composé selon la revendication 1 dans lequel R₂ est hydroxy.

3. Composé selon la revendication 1 ou 2, dans lequel R₁ est méthoxy.

4. Composé selon l'une quelconque des revendications précédentes dans lequel R₃ est méthoxy.

5. Composé selon l'une quelconque des revendications 1 à 4 qui est la 3'-désamino-3'',4'-anhydro-[2''(S)-méthoxy-3''(R)-hydroxy-4''-morpholinyl]doxorubicine.

6. Procédé de préparation d'un composé tel que défini dans la revendication 1, qui comprend
(a) la réaction d'un dérivé N-oxyde d'anthracycline de formule 4 : 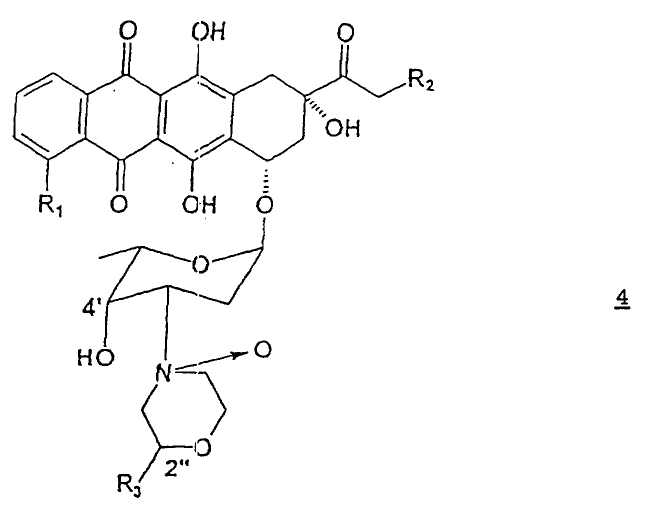 dans laquelle R₁, R₂, R₃ et la configuration en C-2'' sont tels que définis dans la revendication 1, avec un sel de fer (II) en présence d'un agent complexant du fer, et
(b) si nécessaire, la conversion du dérivé morpholinyle résultant de formule 2 en un sel pharmaceutiquement acceptable de celui-ci.

7. Procédé selon la revendication 6 dans lequel l'étape (a) est effectuée dans de l'eau, le sel de fer est le chlorure de fer (II) tétrahydraté et l'agent complexant est l'acide tartrique.

8. Composition pharmaceutique comprenant un diluant ou un véhicule pharmaceutiquement acceptable et, en tant que principe actif, un dérivé morpholinyle de formule (2) ou un sel pharmaceutiquement acceptable de celui-ci, tel que défini dans l'une quelconque des revendications 1 à 5.

9. Dérivé morpholinyle de formule 2 ou sel pharmaceutiquement acceptable de celui-ci, tel que défini dans l'une quelconque des revendications 1 à 5 pour utilisation dans une méthode de traitement par thérapie du corps humain ou animal.

10. Dérivé morpholinyle de formule 2 ou sel pharmaceutiquement acceptable de celui-ci, tel que défini dans l'une quelconque des revendications 1 à 5 pour utilisation en tant qu'agent antitumoral.
